# EUROPÄISCHE PATENTSCHRIFT

(11) **EP 2 816 979 B1**
(45) Veröffentlichungstag und Bekanntmachung des Hinweises auf die Patenterteilung: **22.06.2016**
(21) Anmeldenummer: 13706924.1
(22) Anmeldetag: 22.02.2013
(51) Int. Cl.: A61F 2/64, A61F 2/68, A61F 2/72, A61F 2/76, A61F 5/01

(54) **VERFAHREN ZUR STEUERUNG EINES KÜNSTLICHEN ORTHESEN- ODER PROTHESENKNIEGELENKES**
METHOD FOR CONTROLLING AN ARTIFICIAL ORTHOTIC OR PROSTHETIC KNEE JOINT
PROCÉDÉ DE COMMANDE D'UNE ARTICULATION DE GENOU ARTIFICIELLE D'UNE ORTHÈSE OU PROTHÈSE

(30) Priorität: 22.02.2012 DE 102012003369
(43) Veröffentlichungstag der Anmeldung: 31.12.2014
(73) Patentinhaber: Otto Bock HealthCare GmbH, 37115 Duderstadt (DE)
(72) Erfinder: ALBRECHT-LAATSCH, Erik, 37124 Rosdorf (DE)
(74) Vertreter: Stornebel, Kai
(86) Internationale Anmeldenummer: PCT/EP2013/000519
(87) Internationale Veröffentlichungsnummer: WO 2013/124071

(56) Entgegenhaltungen:
- EP-B1- 1 237 513
- WO-A1-2006/069264
- WO-A1-2010/129716
- DE-A1-102008 027 639
- DE-A1-102009 052 895
- DE-A1-102009 056 466
- US-A1- 2004 267 379

## Beschreibung

Die Erfindung betrifft ein Verfahren zur Steuerung eines künstlichen Orthesen- oder Prothesenkniegelenkes, an dem eine Unterschenkelkomponente angeordnet und der eine Widerstandseinrichtung mit zumindest einem Aktuator zugeordnet ist, über den der Beugewiderstand in Abhängigkeit von Sensordaten verändert wird, die während der Benutzung des Orthesen- oder Prothesenkniegelenkes über zumindest einen Sensor ermittelt werden.

Prothesen- oder Orthesenkniegelenke ersetzen oder unterstützen die Funktion eines natürlichen Kniegelenkes. Um eine möglichst optimale Funktionalität des -künstlichen Kniegelenkes zu erreichen, sind eine Vielzahl von Konstruktionen auf dem Markt, die das Verhalten der Kniegelenke während der Standphase und der Schwungphase beeinflussen. Mechatronische Kniegelenke sind bekannt, bei denen über mehrere verschiedene Sensoren die Bewegungssituationen erfasst werden und auf Grundlage der Sensordaten eine Widerstandseinrichtung angesteuert wird, über die der Beugewiderstand oder der Streckwiderstand variiert wird. Eine Grundproblematik besteht darin, dass die große Vielfalt der möglichen Bewegungssituationen sich nur schwer in einfache Regeln fassen lässt. Daher werden zur Steuerung von Aktuatoren und Bremsen sogenannte Zustandsmaschinen eingesetzt, die hochkomplex sind und viele verschiedene Aktivitäten abbilden. Nachteilig hieran sind der lange Entwicklungszeitraum und der Einsatz aufwendiger Bauteile.

Die DE 10 2009 052 895 A1 betrifft ein Verfahren zur Steuerung eines künstlichen orthetischen oder prothetischen Gelenkes einer unteren Extremität mit einer Widerstandseinrichtung. Der Widerstandseinrichtung ist zumindest ein Aktuator zugeordnet, über den der Beuge- und/oder Streckwiderstand in Abhängigkeit von Sensordaten verändert wird, wobei während der Benutzung des Gelenkes über Sensoren Zustandsinformationen bereitgestellt werden. Der Beugewiderstand wird in der Standphase erhöht oder nicht verringert, wenn ein in Richtung auf die Vertikale abnehmender Inertialwinkel eines Unterschenkelteils und ein gleichzeitig belasteter Vorfuß ermittelt werden.

Die EP 1 237 513 B1 betrifft eine die Existenz oder Funktion einer Gliedmaße ersetzende Unterstützungsvorrichtung aus mindestens zwei, durch ein künstliches Gelenk miteinander verbundenen Teilen und einer Kontrollvorrichtung. Die Unterstützungsvorrichtung umfasst einen Sensor, der einen Neigungswinkel bezogen auf eine Schwerkraftlinie eines mit dem Gelenk verbundenen Teils erfasst und an die Kontrollvorrichtung gekoppelt ist. Die Kontrollvorrichtung ist so angeordnet, dass sie das Gelenk auf der Basis von vom Sensor übermittelten Neigungswinkeldaten beeinflusst. Der Neigungswinkelsensor ist bei einer Ausgestaltung als Prothesenkniegelenk an einem Rohrschenkel angeordnet, zur Ergänzung der Datensituation kann ein zweiter Sensor an dem Unterschenkel angeordnet sein.

Die DE 10 2008 027 639 A1 betrifft ein Orthesengelenk zum Unterstützen eines anatomischen Kniegelenks mit einem Gelenkoberteil und einem Gelenkunterteil, die gelenkig miteinander verbunden sind. Ein Sperrelement zum automatischen Entriegeln und Verriegeln des Orthesengelenkes in einer beliebigen Position ist vorgesehen, ebenso wie ein Betätigungselement für das Sperrelement und ein Sensormittel zum Erfassen relevanter Informationen für das Entriegeln und Verriegeln. Eine Auswerteeinheit zum Auswerten der erfassten Informationen und zum Weiterleiten dieser Information an eine Steuerungs- und/oder Regelungseinheit für das Betätigungselement ist ebenfalls vorhanden. Das Sensormittel weist mindestens zwei Sensoren aus der Gruppe Neigungssensoren, Drehwinkelsensoren, Beschleunigungssensoren oder Gyroskope zum Erfassen von Informationen auf, die den Bewegungs- und/oder Ruhezustand eines Menschen beschreiben. Es können zwei Sensoren eines Typs oder je ein Sensor unterschiedlicher Typen ausgewählt werden. Alle Sensoren sind von dem anatomischen Gelenk, insbesondere Kniegelenk, abwärts angeordnet.

Die WO 2010/129716 A1 betrifft ein Verfahren zur Steuerung einer verstellbaren prothetischen oder orthetischen Vorrichtung, bei dem über einen geomagnetischen Sensor eine Vielzahl von Datenpunkten über ein Zeitintervall aufgenommen wird, aus der Lagedaten einer prothetischen oder orthetischen Vorrichtung in Bezug auf das Magnetfeld der Erde bereitgestellt werden. Die Vielzahl von Datenpunkten wird über das Zeitintervall ausgewertet, indem die Lagedaten mit vordefinierten Mustern unsicheren Gehens verglichen werden. Wenn die Lagedaten mit einem der vordefinierten Muster unsicheren Gehens übereinstimmen, werden Steuerungsanweisungen an die prothetische oder orthetische Vorrichtung ausgegeben.

Die DE 10 2009 056 466 A1 betrifft ein Verfahren zur adaptiven Steuerung und Regelung einer Prothese mit Willkürsteuerung, wobei zunächst an einem Muskel eines Prothesenträgers eine Vielzahl an Muskelaktivitätsignalen gemessen wird. Gleichzeitig werden Zustandsinformationen zu einem tatsächlichen Bewegungszustand des Prothesenträgers gemessen, aus welchen mindestens ein möglicher, aktueller Bewegungszustand bestimmt wird. Danach werden aus den Muskelaktivitätssignalen mit Hilfe eines Verfahrens zur Detektion von Signalmustern Muskelaktivitätsmerkmale extrahiert. Anschließend wird aus den extrahierten Muskelaktivitätsmerkmalen mit Hilfe des mindestens einen möglichen, aktuellen Bewegungszustandes, mindestens ein Willkürsignal bestimmt und das Willkürsignal zur Auswertung und/oder zur Regelung und/oder Steuerung einer Aktorik der Prothese verwendet.

Die US 2004/267379 A1 betrifft eine Vorrichtung zum Messen der relativen Winkellage eines ersten und eines zweiten Körpers in Bezug auf einen Punkt. Die Vorrichtung umfasst ein erstes Messeelement und ein zweites Messelement, die relativ zueinander bewegbar und mit dem ersten Körper bzw. dem zweiten Körper verbindbar sind. Das erste Messelement umfasst einen ersten Neigungssensor, der eine erste Detektionsachse aufweist und ein erstes Neigungssignal bereitstellt, welches mit einem ersten Neigungswinkel der ersten Detektionsachse in Bezug auf eine Referenzachse korreliert. Das zweite Messelement umfasst einen zweiten Neigungssensor, der eine zweite Detektionsachse aufweist und ein zweites Neigungssignal bereitstellt, welches mit einem zweiten Neigungswinkel der zweiten Detektionsachse in Bezug auf die Referenzachse korreliert.

Die WO 2006/069264 A1 betrifft ein System zur Analyse der Bewegung einer mit einem Stumpf verbundenen Vorrichtung. Das System umfasst zumindest einen Beschleunigungsaufnehmer, der eingerichtet ist, einen Zeitverlauf von Bewegungssignalen zu generieren, der auf eine Bewegung der mit einem Stumpf verbundenen Vorrichtung hindeutet. Ein Bearbeitungsmodul kommuniziert dabei mit dem zumindest einen Beschleunigungsaufnehmer. Das Bearbeitungsmodul umfasst einen Filter, der eingerichtet ist, den vom zumindest einen Beschleunigungssensor ausgehenden Zeitverlauf des Bewegungssignals zu verarbeiten und der dazu eingerichtet ist, eine Vielzahl von gefilterten Signalen zu generieren. Das Bearbeitungsmodul umfasst ein Vergleichsmodul, das eingerichtet ist, die Vielzahl der gefilterten Signale mit einer Vielzahl von Mustersignalen zu vergleichen, und das eingerichtet ist, Stumpfbewegungen aus der Bewegung der mit dem Stumpf verbundenen Vorrichtung zu identifizieren.

Aufgabe der vorliegenden Erfindung ist es, ein Verfahren zur Steuerung eines künstlichen Orthesen- oder Prothesenkniegelenkes bereitzustellen, mit dem auf eine einfache und kostengünstige Art und Weise ein sicheres und angenehmes Gangverhalten der Prothese oder Orthese erzielt werden kann.

Erfindungsgemäß wird diese Aufgabe durch ein Verfahren mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Das Verfahren zur Steuerung eines künstlichen Orthesen- oder Prothesenkniegelenkes, an dem eine Unterschenkelkomponente angeordnet und der eine Widerstandseinrichtung mit zumindest einem Aktuator zugeordnet ist, über den der Beugewiderstand in Abhängigkeit von Sensordaten verändert wird, die während der Benutzung des Orthesen- oder Prothesenkniegelenkes über zumindest einen Sensor ermittelt werden, sieht vor, dass ausschließlich über zumindest einen Inertialsensor der Absolutwinkel der Unterschenkelkomponente ermittelt und der ermittelte Winkel mit zumindest einem Schwellwert verglichen und bei Erreichen, insbesondere Überschreiten des Schwellwertes der Beugewiderstand verändert, insbesondere verringert wird. Dadurch kann auf Sensordaten für einen Steueralgorithmus verzichtet werden, die unterschiedliche Messgrößen erfassen, beispielsweise ein Drehmoment, eine Kraft und einen Winkel, die dann relativ aufwendig verarbeitet werden müssen. Die Beschränkung auf die Verwendung von Absolutwinkeln, die über einen oder mehrere Inertialsensoren gemessen werden, wobei der Absolutwinkel der Unterschenkelkomponente ermittelt wird, ist eine einfache und gleichzeitig überraschend zuverlässige Art und Weise, wie eine Steuerung einer Widerstandsänderung bei einer Widerstands- oder Dämpfungseinrichtung bei einem künstlichen Orthesen- oder Prothesenkniegelenk realisiert werden kann. Es ist dabei auch vorgesehen, dass mehrere Schwellwerte festgelegt werden, die bei Erreichen oder bei Über- oder Unterschreiten eine Anpassung des Beugewiderstandes auslösen. Dadurch kann eine Dämpfungskurve erreicht werden, die an den Patienten angepasst ist und ein harmonisches Gangbild ermöglicht.

Eine Weiterbildung der Erfindung sieht vor, dass eine Winkelgeschwindigkeit der Unterschenkelkomponente aus den Sensordaten der Inertialsensoren berechnet und der Beugewiderstand nur dann verringert wird, wenn die Winkelgeschwindigkeit ungleich Null ist, also die Unterschenkelkomponente sich in Extensions- oder Flexionsrichtung bewegt. Auf diese Weise wird sichergestellt, dass nur während des Gehens in Abhängigkeit von dem Absolutwinkel der Unterschenkelkomponente eine Widerstandsänderung durchgeführt wird. Die Kombination von Absolutwinkel mit der Winkelgeschwindigkeit zur Detektion einer Schwungphase und damit zur Festlegung des Zeitpunktes, wann ein Beugewiderstand von einer Standphasendämpfung auf eine Schwungphasendämpfung verringert wird, hat sich auch bei langsamen Gehgeschwindigkeiten als zuverlässig herausgestellt. Auch ist es möglich, durch die Kombination von Absolutwinkel und Winkelgeschwindigkeit andere Phasen der Bewegung während des Gehens zuverlässig zu ermitteln, so dass der Beugewiderstand oder auch der Steckwiderstand in einem Umfang verändert werden kann, dass ein harmonisches Gangbild entsteht und der Patient eine sichere und wirkungsvolle Unterstützung erhält.

Eine Weiterbildung der Erfindung sieht vor, dass der Absolutwinkel ausschließlich über einen oder mehrere Inertialsensoren ermittelt wird, der oder die an der Unterschenkelkomponente oder an einer distal daran befestigten Orthesen- oder Prothesenkomponente befestigt ist oder sind. Die distal angeordneten Orthesen- oder Prothesenkomponenten sind insbesondere Prothesenfüße oder Bügel oder Stützschalen für einen natürlichen Fuß bei einer Orthese. Der Sensor oder die Sensoren werden bevorzugt medial und/oder lateral an der jeweiligen Unterschenkelkomponente befestigt, um den Absolutwinkel der Unterschenkelkomponente zu ermitteln, also dessen Position zu einer festen Bezugsgröße, insbesondere der Vertikalen.

Der Absolutwinkel kann über 2D- oder 3D-Magnetfeldsensoren, 2D- oder 3D-Beschleunigungssensoren und/oder 1 D-, 2D- oder 3D-Gyroskope ermittelt werden. Bei einem Einsatz mehrerer Sensoren können die Sensordaten mehrerer Inertialsensoren miteinander fusioniert werden, um auf der Grundlage mehrerer, verschiedener Sensorsignale die tatsächliche Orientierung der Unterschenkelkomponente sicher feststellen zu können. Messungenauigkeiten oder Störungen bei einem Sensor können dann durch den oder die anderen Sensoren ausgeglichen werden.

Der Schwellwert des Absolutwinkels der Unterschenkelkomponente kann auf den Wert eingestellt werden, den die Unterschenkelkomponente am Ende der Standphase einnimmt, so dass durch die individuelle Einstellung auf besondere körperliche Eigenschaften des Patienten Rücksicht genommen werden kann. Ebenso kann auf bevorzugte Bewegungsmuster Rücksicht genommen werden, indem der Schwellwert des Absolutwinkels von dem zuständigen Orthopädietechniker individuell an das Gangmuster des Patienten angepasst wird. Als das Ende der Standphase wird derjenige Zeitpunkt innerhalb eines Gangzyklusses verstanden, an dem die Fußspitze beim Abrollen des Fußes gerade noch den Boden berührt, unmittelbar bevor die Fußspitze den Kontakt zu dem Boden verliert und angehoben wird. Üblicherweise beugt dann das Kniegelenk weiter ein, so dass eine Flexion im Kniegelenk den Abstand der Fußes von dem Boden vergrößert.

Der Beugewiderstand kann zwischen zwei festgelegten Werten umgeschaltet werden, so dass ein geringer Beugewiderstand während der Schwungphase und ein hoher Beugewiderstand während der Standphase oder in einem Notfallmodus vorhanden ist. Ein solcher Notfall kann bei einem Sturz oder bei einem Stolpern aktiviert werden. Dann kann ein erhöhter Beugewiderstand vorgesehen sein, um ein ungebremstes Einbeugen im Knie zu verhindern. Die Auslegung des Aktuators ist vorteilhafterweise dergestalt gewählt, dass bei einem anliegenden Flexionsmoment keine Auslösung durch den Aktuator stattfinden kann, um die Dämpfung zu verringern. Dies kann beispielsweise dadurch erreicht werden, dass die Leistung des Aktuators kleiner als die Leistung bemessen wird, die notwendig ist, um den Gegendruck eines mit einem Flexionsmoment belasteten Prothesengelenk zu überwinden. Auf diese Weise wird eine Betätigungssperre des Aktuators aufgrund der leistungstechnischen oder mechanischen Auslegung erreicht, so dass keine zusätzlichen Steuerungen oder Sensoren notwendig sind. Befindet sich der Prothesennutzer in einer Position mit einem eingebeugten Gelenk, das belastet ist, kann aufgrund der gewählten geringen Leistung des Aktuators keine Verstellung gegen den Innendruck des Systems erfolgen, wodurch ein Öffnen der Dämpfung und ein spontanes Absenken der Dämpfung in potentiell gefährlichen Situationen vermieden werden kann, auch wenn die vorgegebenen anderen Parameter zur Änderung der Flexionsdämpfung erfüllt sind.

Eine Weiterbildung der Erfindung sieht vor, dass der Beugewiderstand verändert, insbesondere erhöht wird, wenn die Winkelgeschwindigkeit einen Nullpunkt erreicht hat und eine Umkehr der Bewegungsrichtung der Unterschenkelkomponente ermittelt wird. Wird der Beugewiderstand am Ende der Schwungphasenflexion erhöht, wird dadurch eine erhöhte Sicherheit gegen ein ungewolltes Einbeugen bei einem Anstoßen gegen ein Hindernis bereitgestellt. Stolpert der Patient, ermöglicht eine Erhöhung des Beugewiderstandes, dass der Patient sich über das mit der Orthese oder Prothese versorgte Bein abstützen kann, ohne dass das Kniegelenk einbeugt. Wird am Ende der Schwungphase die Umkehrung der Bewegungsrichtung ermittelt, kann der Beugewiderstand verringert werden, um die anfängliche Standphasenflexion zuzulassen. Die Umkehrung der Bewegungsrichtung am Ende der Schwungphase kann dadurch ermittelt werden, dass die Winkelgeschwindigkeit einen Nullpunkt erreicht und der Absolutwinkel sich verringert.

Aus den Inertialsensordaten kann auch die Winkelbeschleunigung der Unterschenkelkomponente ermittelt werden, wobei vorgesehen ist, dass bei Überschreiten eines Schwellwertes der Beugewiderstand erhöht oder nicht verringert wird. Dies dient zur Erkennung von Sondersituationen, beispielsweise beim Stolpern. Die Winkelbeschleunigung wird bevorzugt beim Vorwärtsgehen ermittelt, so dass die häufigsten Gehsituationen erfasst und berücksichtigt werden können. Durch das Überschreiten der Winkelbeschleunigung über einen Grenzwert kann angezeigt werden, dass die harmonische Gehbewegung unterbrochen ist, so dass ein anderer Dämpfungswert als der der Schwungphasendämpfung sinnvoll erscheint. In der Regel ist dann die Flexionsdämpfung zu erhöhen oder auf einem hohen Wert zu belassen.

Nachfolgend wird ein Ausführungsbeispiel der Erfindung anhand der Figuren näher erläutert. Es zeigen:
- Figur 1 -: eine schematische Darstellung einer Protheseneinrichtung;
- Figur 2 -: ein Ablaufdiagramm der Steuerung; sowie
- Figur 3 -: eine Unterschenkelwinkeldiagramm.

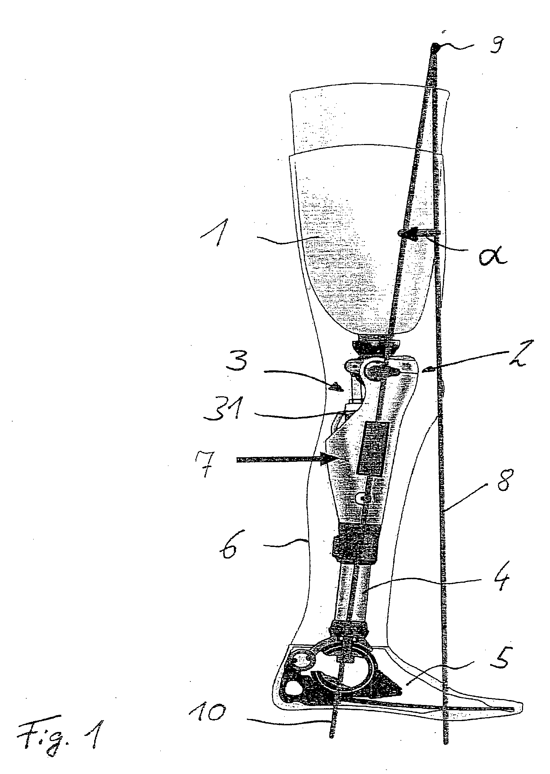

In der Figur 1 ist in einer schematischen Darstellung ein Prothesenbein mit einem Prothesenschaft 1 zur Aufnahme eines Oberschenkelstumpfes und zur Festlegung des Prothesenbeins an einem Patienten vorgesehen. An dem distalen Ende des Prothesenschaftes 1 ist ein Prothesenkniegelenk 2 angeordnet, das mit einer Widerstandseinrichtung 3 beispielsweise in Gestalt eines Hydraulikdämpfers oder einer Schlingfederbremse ausgestattet ist. An dem distalen Ende des Prothesenkniegelenkes 2 sind ein Unterschenkelrohr 4 und ein Prothesenfuß 5 als weitere distale Komponenten vorgesehen. Die Funktionselemente aus Prothesenschaft 1, Prothesenkniegelenk 2, Unterschenkelrohr 4 sowie Prothesenfuß 5 sind durch eine Kosmetik 6 umhüllt, um einen möglichst natürlichen Gesamteindruck zu vermitteln.

An der Unterschenkelkomponente, die aus dem distalen Teil des Prothesenkniegelenkes 2, dem Unterschenkelrohr 4 und dem Prothesenfuß 5 besteht, ist im dargestellten Ausführungsbeispiel ein Inertialsensor 7 als Winkelaufnehmer angeordnet. Der Inertialsensor 7 kann als Magnetfeldsensor, Beschleunigungssensor oder Gyroskop ausgebildet sein. Es ist auch möglich, dass mehrere Inertialsensoren 7 an der Unterschenkelkomponente angeordnet sind, beispielsweise zusätzlich zu der Anbringung an dem distalen Teil des Prothesenkniegelenkes 2 an dem Unterschenkelrohr 4 oder dem Prothesenfuß 5. Die Beschleunigungssensoren und Magnetfeldsensoren können als 2D- oder 3D Sensoren ausgebildet sein, zur Ermittlung von Gyroskopdaten kann ein Gyroskop als 1D oder 2D - oder 3D- Gyroskop ausgebildet sein. Es können mehrere Inertialsensoren des gleichen Typs an der Unterschenkelkomponente angeordnet sein, ebenso können Inertialsensoren 7 unterschiedlichen Typs, beispielsweise Beschleunigungssensor und Gyroskop, an der Unterschenkelkomponente festgelegt sein.

Der Inertialsensor 7, der als Winkelsensor ausgebildet ist, ermittelt den Winkelwert der Unterschenkelkomponente relativ zu einer Schwerpunktlinie 8, die durch ein Zentrum 9 der Schwerkraft verläuft. Das Zentrum 9 der Schwerkraft entspricht dem Körperschwerpunkt des Patienten, und der Winkel α wird zwischen der Schwerpunktlinie 8 und der Längserstreckung der Unterschenkelkomponente durch den Körperschwerpunkt 9 in der gestreckten Stellung des Prothesenkniegelenkes 2 am Ende der Standphase ermittelt. Die Orientierung der Unterschenkelkomponente in der Figur 1 ist durch die Verbindungsgerade 10 entlang der Längserstreckung des Unterschenkelrohres 4 durch die Schwenkachse des Prothesenkniegelenkes 2 definiert. In der dargestellten Position befindet sich das Prothesenkniegelenk 2 in der Extensionslage am Ende der Standphase. Der hier vorhandene Winkel α der Unterschenkelkomponente relativ zu der Schwerpunktlinie 8 ist als Schwellwert gespeichert. Bei diesem Wert liegen eine Extension des Prothesenkniegelenkes 2 sowie des Hüftgelenkes und damit eine Schrittrücklage des Beines vor, und es ist für den Anwender sicher, die Schwungphase einzuleiten. Ist gleichzeitig ein Abrollvorgang des Prothesenfußes 5 nach anterior detektierbar, was sich in einer Winkelgeschwindigkeit α' > 0 und der Winkelzunahme niederschlägt, liegt ein Schritt ebenfalls nach anterior vor. Aufgrund dieser Daten wird ein Aktuator 31 der Widerstandseinrichtung 3 des Prothesenkniegelenkes 2 dergestalt aktiviert, dass eine vorhandene Standphasendämpfung verringert wird und so lange niedrig gehalten bleibt, bis die Winkelgeschwindigkeit α' in der mittleren Schwungphase einen Nullpunkt hat. Anhand von Erfahrungswerten kann ein Winkelbereich für die Position der Unterschenkelkomponente am Ende der Schwungphase eines normalen Schrittes hinterlegt sein. Wird kein vollständiger Schritt ausgeführt, also der Winkelbereich nicht erreicht oder wird eine nicht harmonische Veränderung der Winkelgeschwindigkeit α' festgestellt, wird über den Aktuator 31 die Widerstandseinrichtung 3 dergestalt beeinflusst, dass ein erhöhter Beugewiderstand vorliegt. Wird der unterbrochene Schritt fortgesetzt, kann erneut eine Absenkung des Flexionswiderstandes ausgeführt werden, um den Schritt zu beenden.

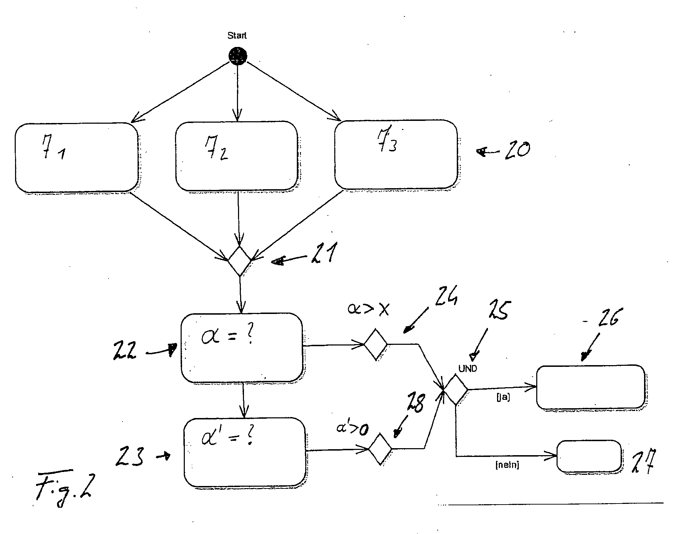

In Figur 2 ist die Funktionsweise der Steuerung als Schaubild dargestellt. Nach dem Start der Steuerung wird der jeweilige Sensorwert in einem ersten Schritt 20 ermittelt. Es können mehrere Sensorwerte 71, 72, 73 über die Inertialsensoren 7 gemessen werden. Neben der Möglichkeit, drei Sensorwerte 71, 72, 73 der Unterschenkelkomponente zu bestimmen, beispielsweise durch drei unterschiedliche Inertialwinkelsensoren 7 wie Magnetfeldsensor, Beschleunigungssensor und Gyroskop, besteht auch die Möglichkeit, mehrere Sensorwerte durch mehrere gleichartige Inertialsensoren 7 zu bestimmen. Grundsätzlich ist es auch möglich, nur mit einem Inertialsensor 7 den Sensorwert zu erfassen.

Die von den Inertialsensoren 7 erfassten Daten werden in einem weiteren Arbeitsschritt 21 fusioniert, um Ungenauigkeiten auszugleichen und um eine möglichst vollständige Datenlage zur Berechnung des Absolutwinkels α zu haben. Sofern nur ein Inertialsensor 7 vorgesehen ist, müssen die Daten nicht fusioniert werden.

In einem nachgelagerten Auswerteschritt 22 wird aus den Sensordaten 71, 72, 73 der Winkel α der Unterschenkelkomponente zur Schwerkraftlinie 8 berechnet. Ebenfalls ist es möglich, parallel dazu in einem weiteren Arbeitsschritt 23 die Winkelgeschwindigkeit α' der Unterschenkelkomponente zu berechnen.

Der durch beispielsweise einen Kalmanfilter berechnete Winkel α zur Schwerkraftlinie 8 wird dann in einem weiteren Schritt 24 mit einem Schwellwert X verglichen, der vorab festgelegt wurde. Sobald der Absolutwinkel α größer als der voreingestellte Schwellwert X ist, kann in einem Steuerungsfall, bei dem die Winkelgeschwindigkeit α' nicht berücksichtigt wird, der Aktuator 31 in einem weiteren Schritt 26 so aktiviert werden, dass die Dämpfungseinrichtung 3 einen verringerten Widerstand zur Einleitung der Schwungphase einnimmt. Wird der Schwellwert nicht erreicht, wird der Aktuator 31 im Alternativschritt 27 nicht entsprechend betätigt und der Widerstand der Widerstandseinrichtung 3 bleibt unverändert.

Wird zusammen mit dem Winkel α auch die Winkelgeschwindigkeit α' im Schritt 23 errechnet und ist die Winkelgeschwindigkeit α' größer als Null, so wird eine Gehbewegung im Schritt 28 detektiert. In einem Zusammenführungsschritt 25 werden der Winkel α und die Winkelgeschwindigkeit α' miteinander gekoppelt, liegen beide Schwellwerte vor oder werden überschritten, wird der Aktuator gemäß Schritt 26 aktiviert, liegt einer der beiden Schwellwerte für den Winkel α oder die Winkelgeschwindigkeit α' nicht vor, wird der Aktuator 31 gemäß Schritt 27 nicht aktiviert.

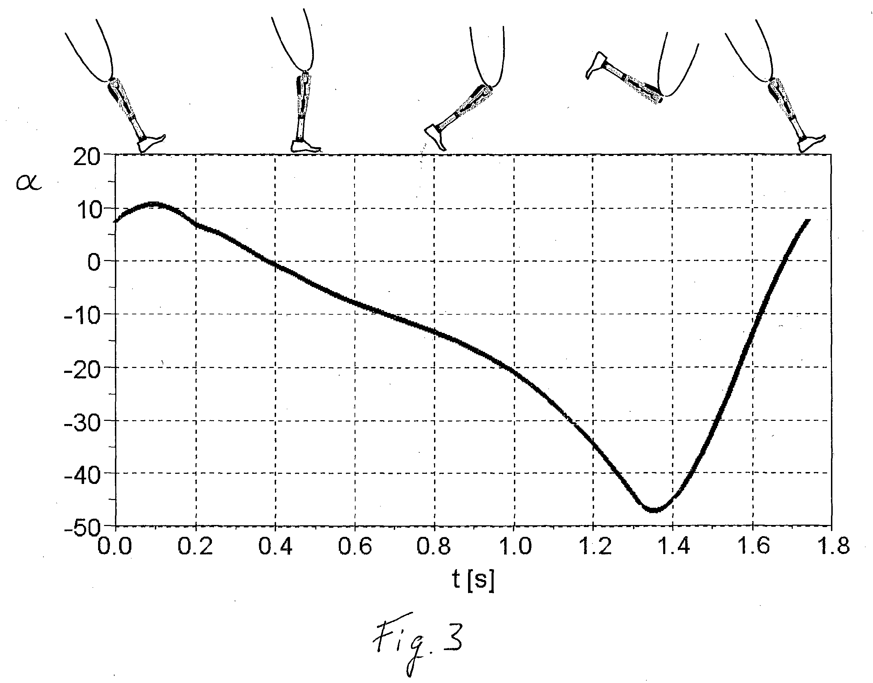

In der Figur 3 ist ein Diagramm des Unterschenkelwinkels α über die Zeit dargestellt. Oberhalb des Diagramms sind die einzelnen Positionen während eines Schrittzyklusses dargestellt. Der Schritt beginnt mit dem Aufsetzen der Ferse, dem sogenannten Fersenstoß, wodurch zunächst der Unterschenkelwinkel α leicht erhöht wird. Im Verlauf des weiteren Schrittes wird der Prothesenfuß vollständig aufgesetzt und der Unterschenkel bzw. die Unterschenkelkomponente erreicht nach ungefähr 0,4 Sekunden die Senkrechte, so dass der Unterschenkelwinkel α 0° beträgt. Durch die Bewegung weiter nach vorne wird der Unterschenkel bzw. die Unterschenkelkomponente weiter gekippt, so dass der Unterschenkel α sich betragsmäßig vergrößert. Bei ca. 0,8 Sekunden ist das Ende der Standphase erreicht, die Prothesenfußspitze verlässt den Boden, so dass eine Schwungphasenflexion eintritt. Am Ende der Schwungphase ist beim normalen Gehen bei einem Unterschenkelwinkel von 45° der Umkehrpunkt erreicht. Dies ist bei ungefähr 1,3 Sekunden der Fall. Anschließend erfolgt eine Bewegungsumkehr, der Unterschenkelwinkel α verringert sich betragsmäßig und nähert sich der Vertikalen an, bis er bei ca. 1,7 Sekunden wieder die Vertikale erreicht und dann durch das nach vorne strecken des Fußes weiter relativ zu der Vertikalen verkippt wird, diesmal jedoch in den positiven Winkelbereich hinein. Über den Verlauf des Unterschenkelwinkels α können mehrere Schwellwerte für diesen Unterschenkelwinkel α festgelegt werden. Werden diese Schwellwerte erreicht oder überschritten bzw. unterschritten, je nachdem, von welcher Richtung man auf den Schwellwert schaut, kann die Veränderung des Beugewiderstandes vorgenommen werden, insbesondere kann nach Erreichen des maximalen Beugewinkels der Beugewiderstand vergrößert werden, um eine Sicherung gegen ein ungewolltes und ungebremstes Einbeugen des Kniegelenkes bei einem Stolpern bereitzustellen. Über die Stellung des Unterschenkels relativ zur Vertikalen kann eine einfache und effektive Steuerung der Dämpfung der Widerstandseinrichtung erfolgen.

Es hat sich herausgestellt, dass der oben beschriebene, einfache Algorithmus gut dazu benutzt werden kann, zwischen einer Standphasendämpfung und einer Schwungphasendämpfung hin und her zu schalten. Besonders zuverlässig ist die Steuerung, wenn der Winkelwert α größer als ein zuvor gespeicherter Schwellwert ist, der durch Betätigung eines Steuerknopfes an der Protheseneinrichtung eingestellt wird, während der Patient in Rückverlagerung steht und die Winkelgeschwindigkeit α' größer als Null ist. Liegen beide Bedingungen vor, kann die Widerstandseinrichtung 3 beispielsweise durch Schalten eines Hydraulikventils oder Lösen einer Sperre einer Bremseinrichtung von einer hohen Standphasendämpfung auf eine geringere Schwungphasenflexionsdämpfung umschalten.

Der Einsatz ausschließlich von Inertialsensoren verringert die Kosten, da die auf Dehnmessstreifen basierenden Momentensensoren für alternative Verfahren sehr teuer sind. Darüber hinaus sind die Inertialwinkelsensoren verschleißfrei.

Neben der ersten Ableitung des Winkelsignals zur Bestimmung der Winkelgeschwindigkeit ist auch die Anwendung der zweiten Ableitung des Winkelsignals möglich, um die Winkelbeschleunigung zu ermitteln. Das Beschleunigungssignal kann zur Sturzdetektion genutzt werden, eine Überschreitung der Winkelbeschleunigung über einen festgelegten Grenzwert kann anzeigen, das die harmonische Gehbewegung unterbrochen ist und ein anderer Dämpfungswert sinnvoller wäre, üblicherweise die Erhöhung des Flexionswiderstandes.

Weiterhin kann durch die Beobachtung der Winkelverläufe auch die Gehgeschwindigkeit geschlossen werden, darüber hinaus kann das Widerstandsverhalten und damit das Dämpfungsverhalten während der Schwungphase nicht nur zwischen zwei Werten binär hin und her geschaltet werden, sondern die Umschaltung kann in zeitlich sehr kleinen Abständen für diskrete Winkelwerte oder für jeden Winkelwert bzw. jede Abtastung des Winkelwertes verstellt werden. Beispielsweise können Hydraulikventile schrittweise verstellt oder Bremseinrichtungen, die ebenfalls als Widerstandseinrichtungen eingesetzt werden können, angepasst eingestellt werden, um ein harmonisches Gangbild zu erzeugen.

Weiterhin kann aus den Winkelverläufen die Widerstandseinrichtung dahingehend eingestellt werden, dass bei einem Fersenauftritt mit einem gestreckten Gelenk eine Standphasenflexion durch Absenkung der Flexionswiderstände ermöglicht wird. Nach dem Auftreten kann auch eine progressive Veränderung der Dämpfung vorgesehen sein, so dass nach dem Absenken einer Standphasenflexion nach dem Fersenauftritt eine stark ansteigende Dämpfung erfolgt, die ein Einknicken nach dem Fersenauftritt bis zu einem bestimmten Winkelwert ermöglicht, darüber hinaus eine weitere Vergrößerung des Winkels durch die Verstellung der Widerstandseinrichtung unterbunden wird.

Die künstlichen Kniegelenke können sowohl als Prothesen, wie im Ausführungsbeispiel dargestellt als auch als Orthesen eingesetzt werden. Die Widerstandseinrichtungen können als einfache Sperren, komplexe Hydrauliken oder Schlingfederbremsen ausgelegt sein. Durch die Berücksichtigung der Winkelgeschwindigkeit α können auch Antriebe genutzt werden, um eine Einbeugung oder Streckung zusätzlich zu ermöglichen. Zur Absicherung gegen ein nicht erwünschtes Einbeugen oder Verringern der Dämpfung unter Last, also bei einem anliegenden Flexionsmoment, kann der Aktuator leistungsmäßig so ausgelegt sein, dass bei einem auftretenden Flexionsmoment die aufzubringende Schaltleistung über der Abgabeleistung des Aktuators liegt, so dass eine Verringerung der Dämpfung und eine damit einhergehende schlagartige Einbeugung nicht erfolgen kann.

## Patentansprüche

1. Verfahren zur Steuerung eines künstlichen Orthesen- oder Prothesenkniegelenkes (2), an dem eine Unterschenkelkomponente (4, 5) angeordnet und der eine Widerstandseinrichtung (3) mit zumindest einem Aktuator (31) zugeordnet ist, über den der Beugewiderstand in Abhängigkeit von Sensordaten verändert wird, die während der Benutzung des Orthesen- oder Prothesengelenkes über einen Sensor (7) ermittelt werden, wobei ausschließlich über zumindest einen Inertialsensor (7) der Absolutwinkel der Unterschenkelkomponente (4, 5) ermittelt wird, **dadurch gekennzeichnet, dass** der ermittelte Winkel mit zumindest einem Schwellwert verglichen und bei Erreichen des Schwellwertes der Beugewiderstand verändert wird.

2. Verfahren nach Anspruch 1, **dadurch gekennzeichnet, dass** eine Winkelgeschwindigkeit der Unterschenkelkomponente (4, 5) aus den Sensordaten des zumindest einen Inertialsensors (7) berechnet und der Beugewiderstand nur dann verringert wird, wenn die Winkelgeschwindigkeit ungleich als Null ist.

3. Verfahren nach Anspruch 1 oder 2, **dadurch gekennzeichnet, dass** der Absolutwinkel ausschließlich über einen oder mehrere Inertialsensoren (7) ermittelt wird, der oder die an der Unterschenkelkomponente (4, 5) oder an einer distal daran befestigten Orthesen- oder Prothesenkomponente befestigt ist oder sind.

4. Verfahren nach einem der voranstehenden Ansprüche, **dadurch gekennzeichnet, dass** der Absolutwinkel über 2D- oder 3D-Magnetfeldsensoren, 2D- oder 3D-Beschleunigungssensoren und/oder 1D- 2D- oder 3D-Gyroskope ermittelt wird.

5. Verfahren nach einem der voranstehenden Ansprüche, **dadurch gekennzeichnet, dass** die Sensordaten mehrerer Inertialsensoren (7) miteinander fusioniert werden.

6. Verfahren nach einem der voranstehenden Ansprüche, **dadurch gekennzeichnet, dass** der Schwellwert des Absolutwinkels der Unterschenkelkomponente (4, 5) auf den Wert eingestellt wird, den die Unterschenkelkomponente (4, 5) am Ende der Standphase einnimmt.

7. Verfahren nach einem der voranstehenden Ansprüche, **dadurch gekennzeichnet, dass** der Beugewiderstand zwischen zwei festgelegten Werten umgeschaltet wird.

8. Verfahren nach einem der voranstehenden Ansprüche, **dadurch gekennzeichnet, dass** der Beugewiderstand verändert wird, wenn die Winkelgeschwindigkeit einen Nullpunkt erreicht hat und eine Umkehr der Bewegungsrichtung der Unterschenkelkomponente (4, 5) ermittelt wird.

9. Verfahren nach einem der voranstehenden Ansprüche, **dadurch gekennzeichnet, dass** die Winkelbeschleunigung der Unterschenkelkomponente (4, 5) ermittelt wird und bei Überschreiten eines Schwellwertes der Beugewiderstand erhöht oder nicht verringert wird.

## Claims

1. Method for controlling an artificial orthotic or prosthetic knee joint (2), on which a lower leg component (4, 5) is arranged and which is provided with a resistance device (3) having at least one actuator (31), by means of which the bending resistance is modified as a function of sensor data which are determined by means of a sensor (7) during use of the orthotic or prosthetic joint, the absolute angle of the lower leg component (4, 5) being determined exclusively by means of at least one inertial sensor (7), **characterized in that** the angle determined is compared with at least one threshold value, and the bending resistance is modified when the threshold value is reached.

2. Method according to Claim 1, **characterized in that** an angular velocity of the lower leg component (4, 5) is calculated from the sensor data of the at least one inertial sensor (7), and the bending resistance is reduced only when the angular velocity is not equal to zero.

3. Method according to Claim 1 or 2, **characterized in that** the absolute angle is determined exclusively by means of one or more inertial sensors (7), which is or are fastened on the lower leg component (4, 5) or on an orthotic or prosthetic component fastened distally thereon.

4. Method according to one of the preceding claims, **characterized in that** the absolute angle is determined by means of 2D or 3D magnetic field sensors, 2D or 3D acceleration sensors and/or 1D, 2D or 3D gyroscopes.

5. Method according to one of the preceding claims, **characterized in that** the sensor data of a plurality of inertial sensors (7) are merged together.

6. Method according to one of the preceding claims, **characterized in that** the threshold value of the absolute angle of the lower leg component (4, 5) is adjusted to the value which the lower leg component (4, 5) adopts at the end of the standing phase.

7. Method according to one of the preceding claims, **characterized in that** the bending resistance is switched between two fixed values.

8. Method according to one of the preceding claims, **characterized in that** the bending resistance is modified when the angular velocity has reached a zero point and reversal of the movement direction of the lower leg component (4, 5) is determined.

9. Method according to one of the preceding claims, **characterized in that** the angular acceleration of the lower leg component (4, 5) is determined, and the bending resistance is increased, or not reduced, when a threshold value is exceeded.

## Revendications

1. Procédé pour la commande d'une articulation de genou artificielle (2) en forme d'orthèse ou de prothèse, sur laquelle est agencé un composant formant jambe (4, 5) et auquel est associé un dispositif à résistance (3) avec au moins un actionneur (31) au moyen duquel la résistance à la flexion est modifiée en fonction de données détectées déterminées au moyen d'un capteur (1) pendant l'utilisation de l'articulation en forme d'orthèse ou de prothèse, dans lequel on détermine exclusivement au moyen d'au moins un capteur inertiel (7) l'angle absolu du composant formant jambe (4, 5),
**caractérisé en ce que** l'angle déterminé est comparé à au moins une valeur seuil et la résistance à la flexion est modifiée lorsqu'on atteint la valeur seuil.

2. Procédé selon la revendication 1, **caractérisé en ce que** l'on calcule une vitesse angulaire du composant formant jambe (4, 5) à partir des données détectées dudit au moins un capteur inertiel (7), et la résistance à la flexion est réduite uniquement quand la vitesse angulaire n'est pas égale à zéro.

3. Procédé selon la revendication 1 ou 2, **caractérisé en ce que** l'angle absolu est déterminé exclusivement au moyen d'un ou plusieurs capteurs inertiels (7) qui est/sont fixé(s) sur le composant formant jambe (4, 5) ou sur un composant distal d'orthèse ou de prothèse fixé à celui-ci.

4. Procédé selon l'une des revendications précédentes, **caractérisé en ce que** l'angle absolu est déterminé au moyen de capteurs de champ magnétique 2D ou 3D, de capteurs d'accélération 2D ou 3D, ou de gyroscopes 1D, 2D ou 3D.

5. Procédé selon l'une des revendications précédentes, **caractérisé en ce que** les données détectées de plusieurs capteurs inertiels (7) sont fusionnées les unes avec les autres.

6. Procédé selon l'une des revendications précédentes, **caractérisé en ce que** la valeur seuil de l'angle absolu du composant formant jambe (4, 5) est réglée à la valeur que le composant formant jambe (4, 5) adopte à la fin de la phase de station debout.

7. Procédé selon l'une des revendications précédentes, **caractérisé en ce que** la résistance à la flexion est commutée entre deux valeurs fixes.

8. Procédé selon l'une des revendications précédentes, **caractérisé en ce que** la résistance à la flexion est modifiée quand la vitesse angulaire a atteint un point zéro et qu'une inversion de la direction de flexion du composant formant jambe (4, 5) est déterminée.

9. Procédé selon l'une des revendications précédentes, **caractérisé en ce que** l'accélération angulaire du composant formant jambe (4, 5) est déterminée et la résistance à la flexion est augmentée ou n'est pas diminuée en cas de dépassement d'une valeur seuil.
